(19) Europäisches Patentamt
European Patent Office
Office européen des brevets

(11) Publication number: **0 146 155**
**B1**

# (12) EUROPEAN PATENT SPECIFICATION

(45) Date of publication of patent specification: **06.04.88**

(51) Int. Cl.⁴: **C 07 D 295/12**, A 61 K 31/40

(21) Application number: **84201573.7**

(22) Date of filing: **01.11.84**

(54) **Ether of N-propanolamine derivative.**

(30) Priority: **11.11.83 GB 8330197**

(43) Date of publication of application:
**26.06.85 Bulletin 85/26**

(45) Publication of the grant of the patent:
**06.04.88 Bulletin 88/14**

(84) Designated Contracting States:
**AT BE CH DE FR GB IT LI LU NL SE**

(56) References cited:
**WO-A-83/02274**
**DE-A-2 802 864**
**US-A-3 962 238**
**US-E- 30 577**

**ULLMANNS "Encyklopädie der technischen Chemie". 4th edition, vol. 17, 1979 "Milchsäure bis Petrolkoks", VERLAG CHEMIE, Weinheim, pages 453-454**

**The file contains technical information submitted after the application was filed and not included in this specification**

(73) Proprietor: **AKZO N.V.**
**Velperweg 76**
**NL-6824 BM Arnhem (NL)**

(72) Inventor: **Winslow, Eileen**
**21 Loch Laxford**
**East Kilbride Lanarshire Scotland (GB)**
Inventor: **Basten, Johannes Egbertus Maria**
**Victorstraat 41**
**NL-6654 AV Afferden (GLD) (NL)**

(74) Representative: **Hermans, Franciscus G.M. et al**
**Postbus 20**
**NL-5340 BH Oss (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 146 155 B1

## Description

The present invention relates to a novel ether of an n-propylamine derivative and acid addition salts thereof, to a method for their preparation and to a pharmaceutical composition containing these compounds as the active principle.

Ethers of n-propanolamine derivatives are known from the U.S. patent specification RE 30.577, where a derivative have been described of the formula:

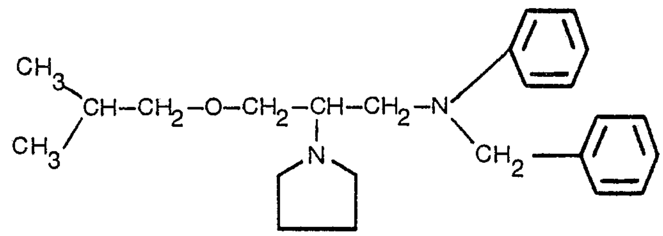

I

This compound of formula I, presently known as bepridil, is effective as a medicament in the treatment of cardiovascular disorders. Its clinical use has, however, in rare cases been attended by a self limiting rhythm disturbance of the so-called ventricular tachycardia type.

It has been noticed that this disturbance predominantly occurs in elderly patients and often is associated with hypokalaemic conditions of the patient, mainly caused by the use of (non $K^+$-sparing) diuretics as co-medication.

This rare arrhythmogenic potential of the compound may nevertheless cause serious adverse effects in certain patients and may limit the general use of this compound, also taking into account that — in the treatment of angina pectoris — diuretics are usually prescribed as co-medication.

The finding of a drug with the same or even better calcium antagonistic activity/anti-anginal activity but without said arrhythmogenic potential would be a definite step forward especially in the safe treatment of certain high risk patient groups such as elderly patients (above 60 years old), patients suffering from hypokalaemic conditions and patients using diuretics as co-medication.

Surprisingly it has now been found in relevant animal tests, that both separate optical isomers of the compound of formula I and especially the R-(dextro-rotatory) isomer do not possess arrhythmogenic activity under specific hypokalaemic conditions in the rat were the racemate exacerbates arrhythmias.

This finding is the more surprising because it suggests that the adverse arrhythmogenic potential of the racemate is merely caused by the combined presence of the dextro- and levo-rotatory isomers in the racemate.

It has, moreover, been found that the further pharmacological profile of either of the two optical isomers does not differ essentially from that of the racemate. Nevertheless the R-(dextro-rotatory) isomer does show a significant increase of calcium-antagonistic activity, (which is an important measure for anti-anginal activity) compared with either the racemate or the levo-rotatory isomer whereas its anti-arrhythmic potency is not significantly different from that of the racemate and the levo-rotatory isomer.

Moreover, the R-isomer does not produce $QT_c$ prolongation. $QT_c$ prolongation is an effect seen in the EEG (electrocardiogram) that is considered less desirable in clinical practice.

In addition, the R-isomer has a much better solubility in water than the racemate, so that this isomer is more suitable especially in various forms of parenteral administration.

These findings all together render the R-isomer (dextro-rotatory isomer) extremely suitable in the treatment of angina pectoris especially under those circumstances (elderly patients, hypokalaemic conditions, co-medication with diuretics) where the racemate is less suitable in view of its arrhythmogenic potential.

The present invention is therefore dealing with the R-(dextro-rotatory)-isomer of the formula:

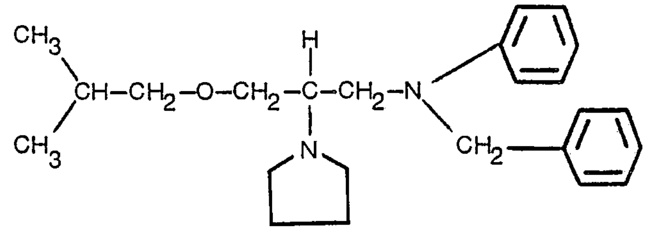

I

and acid addition salts thereof, being substantially free from the corresponding S-iomer(levo-rotatory isomer), with a process for the preparation of this R-isomer and with pharmaceutical preparations containing this R-isomer as the active constituent and which is substantially free from the corresponding S-(levo-rotatory)isomer.

The R-(dextro-rotatory)isomer according to this invention may be prepared by resolution of the racemate using well known methods for resolving racemates. It was found, however, that — using these conventional methods — the yields were fairly low, so that these methods are not suitable for large scale production.

A very suitable method for preparing the R-(dextro-rotatory)isomer according to the invention is starting with the resolution of a racemic compound of the formula II

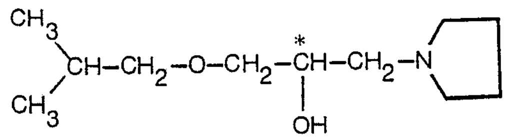

II

(the asterisk indicates the chiral carbon atom). This resolution can be carried out in a conventional manner using well known optically active carboxylic acids.

One of the optically active isomers of formula II (either the S- or the R-isomer) thus obtained is subsequently converted into the R-(dextro-rotatory)isomer of formula I using the following reaction steps:

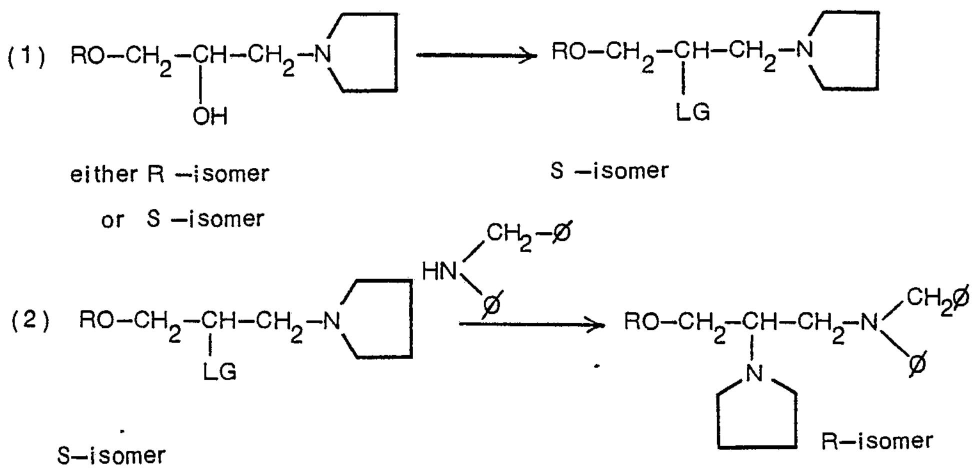

(1)

either R —isomer or S —isomer

S —isomer

(2)

S—isomer

R—isomer

wherein:

LG represents a leaving group, such as halogen or a sulphonyloxy group (e.g. methanesulphonyloxy or p-toluenesulphonyloxy),

R is the isobutyl moiety and ∅ represents phenyl.

The conversion of the hydroxyl group into a leaving group in the first step of the reaction can be carried out with retention of configuration or through a $SN_2$ type reaction mechanism (thus causing inversion) depending on the method selected. For example, by using $SOCl_2$ in toluene as halogenating agent the R-(dextro-rotatory)alcohol of formula II is converted into its S-(levo-rotatory)halogen derivative; and using methanesulphonylchloride the S-(levo-rotatory)-alcohol of formula II is converted into the corresponding S-(levo-rotatory)methanesulphonyloxy derivative. The latter method is the preferred method of preparation.

The condensation reaction (second step) of the (leaving group containing S-isomer) with N-benzylaniline is preferably carried out in the presence of an alkaline substance, such as sodium hydroxide or a quaternary ammonium compound. According to a variant of this condensation N-benzylaniline may first be metallized to its alkali metal derivative with the aid of agents well known to those skilled in the art, such as sodium hydride or sodium, potassium or lithium amide.

Pharmaceutically acceptable acid addition salts of the R-(dextro-rotatory)isomer according to this invention are for example derived from hydrochloric acid, hydrobromic acid, sulphuric acid, methanesulphonic acid, acetic acid, maleic acid, fumaric acid, tartaric acid, ascorbic acid and citric acid. Depending upon the water content of the final reaction mixture, the R-(dextro-rotatory)isomer or its acid addition salt, isolated therefrom, may further be obtained in a hydrated form.

As already said the use of the R-(dextro-rotatory)isomer according to this invention (and acid addition salts thereof) being substantially free from the corresponding S-(levo-rotatory)isomer is extremely advantageous in the treatment of angina pectoris in general but especially in those circumstance where angina pectoris is accompanied with an abnormally low potassium content of the blood (hypokalaemic condition), which for example may be caused by the use of certain diuretics as co-medication.

The R-(dextro-rotatory)isomer according to formula I and acid addition salts thereof may be administered enterally or parenterally, whereby the administration per os and through a continuous infusion into the vein is to be preferred.

Mixed with suitable carriers the compounds of the invention can be processed into a form which is suitable for enteral administration such as pills, tablets, capsules and suppositories. For injection or infusion purposes, the compounds of the present invention are dissolved, emulsified or suspended in a suitable liquid.

The R-(dextro-rotatory)isomer according to this invention is preferably administered in a daily dosage of 0.5—20 mg per kg bodyweight. For oral administration to human beings the preferred daily dosage varies from 50—500 mg and for parenteral administration from 25—1000 mg.

Example 1

1. R-α[(2-methylpropoxy)-methyl]-1-pyrrolidine ethanol

250,0 g of racemic α-[(2-methylpropoxy)-methyl]-1-pyrrolidine-ethanol were dissolved in 1 liter anhydrous ethanol after which 467 g D-(+) dibenzoyl tartaric acid monohydrate was added to the solution.

The precipitated salt crystals, obtained by filtration were then dried and recrystallised 4 times from anhydrous ethanol.

Melting point of the R-(+) salt = 143.3—146.8°C and $[\alpha]_D^{20}$ = +92.3° (c = 0.5 $CH_3OH$).

This dextro-rotatory salt was added to 758 ml of 1 n NaOH, after which the suspension was extracted with ether (3x) and the combined ether extracts washed with 120 ml water (5x). The ether extracts were then dried over magnesium sulphate whereupon the ether solvent was evaporated. The residue was distilled in vacuo.

Yield of the free base: 64.3 gram;
boiling point (0.3 mm): 84—89°C;
$[\alpha]_D^{20}$: + 11.08 (c = 0.5 $CH_3OH$).

2. S-1-[2-chloro-3-(2-methylpropoxy)-propyl]pyrrolidine

64.3 g of the dextro-rotatory isomer obtained in 1. were dissolved in 190 ml anhydrous toluene and the solution was heated up to 40—45°C.

Subsequently a solution of 35 ml thionylchloride in 65 ml toluene was slowly added to the previous solution in about 4 hours while stirring. The reaction mixture was heated to 70—75°C and stirred for 2 hours. The mixture was then cooled down to 0°C whereupon water (about 60 ml) was added very slowly (the temperature was not allowed to rise above 20°C).

The mixture was then poured into ice-water, after which the toluene layer was separated and extracted with water again. To the combined water layers 190 ml toluene were added whereupon the two-layers system was adjusted to pH 9 by adding concentrated ammonia.

The two layers were separated. The toluene layer thus obtained was washed with water dried over magnesium sulphate and evaporated.

Yield: 53.3 g oily substance (75%);
$[\alpha]_D^{20}$ = −24.35° (c = 2 $CH_3OH$);
$R_f$ = 0.43 in methylene chloride:methanol (5:1) on $SiO_2$.

3. R-β[(2-methylpropoxy)-methyl]-N-phenyl-N-(phenylmethyl)-1-pyrrolidine-ethanamine.HCl $H_2O$

A solution of 49.0 g N-benzylaniline in toluene was heated to about 80°C and then slowly added (in about 30 minutes) to 12.7 g sodium amide, suspended in 115 ml anhydrous toluene, while stirring and under nitrogen atmosphere. The mixture was refluxed for two hours and cooled down to about 70°C. Subsequently a solution of 53.3 g of the S-(levo-rotatory)compound (obtained in 2.) in 55 ml toluene was added slowly to the reaction mixture in about ½ hour.

The mixture was refluxed for 2 hours; then it was cooled down to about 10°C, whereupon 55 ml water was slowly added.

The mixture obtained was poured into 220 ml ice-water after which it was stirred for 15 minutes.

The toluene layer was separated from the water layer, after which the toluene layer was washed, dried and evaporated, yielding about 98 grams oily substance.

The oil was purified by chromatographing over a $SiO_2$ column and subsequently over an $Al_2O_3$ column. Yield about 60 g of an oily substance.

The purified oil was then dissolved in about 2000 ml ether. To this solution 80 ml 3.7 n HCl in ethanol was slowly added, after which the solvents were evaporated. The oily residue was subsequently dissolved in moist ethylacetate. After cooling down this solution to about −15°C crystals of the hydrochloride salt were obtained.

Yield: 36.8 g of the HCl salt (containing 1 mol. $H_2O$),
Melting point: 73—74°C,
$[\alpha]_D^{20}$ = +15.6° (c = 1 $CH_2Cl_2$).
Rf in n-butanol:acetic acid:water (4:1:5) = 0.66 on $SiO_2$.

Example 2

1. S-α[(2-methylpropoxy)-methyl]-1-pyrrolidine-ethanol

250,0 of racemic α-[(2-methylpropoxy)-methyl]-1-pyrrolidine-ethanol were dissolved in 1 liter anhydrous ethanol after which 467 g L-[−) dibenzoyl tartaric acid monohydrate was added to the solution.

The precipitated salt crystals, obtained by filtration were then dried and recrystallised from anhydrous ethanol.

Melting point of the S-salt = 136—137°C and $[\alpha]_D^{10} = -93.4°$ (c = 0.54 $CH_3OH$).

This levo-rotatory salt was added to 758 ml of 1 n NaOH, after which the suspenseion was extracted with ether (3×) and the combined ether extracts washed with 120 ml water (5×). The ether extracts were then dried over magnesium sulphate whereupon the ether solvent was evaporated. The residue was distilled in vacuo.

Yield of the free base: 45.6 g;

boiling point (0.3 mm): 83—90°C;

$[\alpha]_D^{20} = 14.0$ (c = 0.77, $CH_3OH$).

2. S-1-[2-methanesulphonyloxy-3-(2-methylpropoxy)-propyl]-pyrrolidine

40.2 g of the S-(−) alcohol obtained in 1. were dissolved in 80 ml anhydrous methylenechloride. To the resulting solution, cooled down to 0°C, 38.1 ml triethylamine were added while stirring. Subsequently 18 ml methanesulphonylchloride, dissolved in 80 ml methylenechloride, were added slowly to the reaction mixture, so that the reaction temperature did not rise above 5°C.

After stirring the reaction mixture for an additional hour at 5°C and 20 hours at room temperature, it is extracted (2×) with a mixture of 100 ml saturated NaCl-solution in water and 100 ml 5% ammonia. The residue is dried on magnesiumsulphate, resulting in 47.0 g of an oily product.

Rf in $CH_2Cl_2/CH_3OH$ (95:5) = 0.47 on $SiO_2$.

3. R-β-[(2-methylpropoxy)-methyl]-N-phenyl-N-(phenylmethyl)-1-pyrrolidine-ethanamine

5.62 g of the "mesylate" obtained in 2. were dissolved in 5 ml anhydrous toluene, after which 6.45 g N-benzylaniline were added. The mixture was stirred under nitrogen at 70°C for 24 hours, and subsequently concentrated. The residue was chromatographed over $SiO_2$, using methylenechloride/methanol (97:3) as solvent yielding 4.4 g of the title product (free base) as an oily product.

The oil was converted into its HCl salt (monohydrate) in the manner described in Example 1.3.

$[\alpha]_D^{20} = +15.6$ (c = 1, $CH_2Cl_2$).

**Claims**

1. The R-(dextro-rotatory)isomer of the formula:

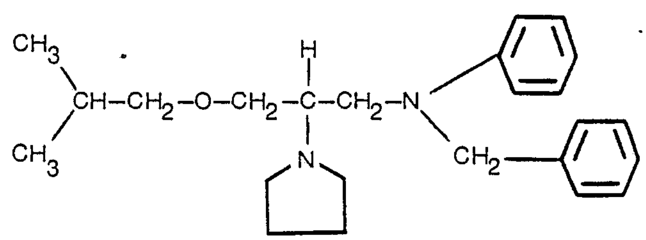

I

and pharmaceutically acceptable acid addition salts thereof, being essentially free from the corresponding S-(levo-rotatory)isomer.

2. A pharmaceutical preparation containing as the active principle the R-(dextro-rotatory)isomer described in claim 1 or a pharmaceutically acceptable acid addition salt thereof, which preparation is essentially free from the corresponding S-(levo-rotatory)isomer.

3. An anti-anginal pharmaceutical preparation according to claim 2 for administration to elderly patients, patients under hypokalaemic conditions, or patients using non $K^+$-sparing diuretics as co-medication.

4. Use of a compound according to claim 1 for the manufacture of a medicament for the treatment of angina pectoris.

5. Use of a compound according to claim 1 for the manufacture of a medicament for the treatment of angina pectoris in patients having low potassium contents of the blood or in patients using non $K^+$ sparing diuretics as co-medication.

**Patentansprüche**

1. Das (rechtsdrehende) R-Isomere der Formel

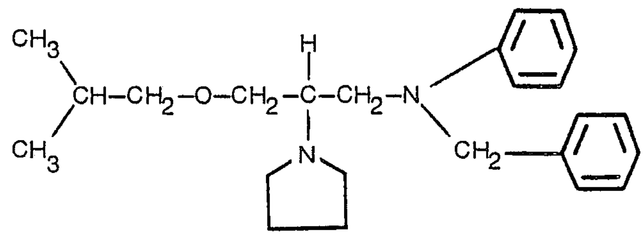

I

und dessen pharmazeutisch unbedenkliche Säureadditionssalze, im wesentlichen frei von dem entsprechenden (linksdrehenden) S-Isomeren.

2. Pharmazeutisches Präparat, das als Wirkstoff das (rechtsdrehende) R-Isomere nach Anspruch 1 oder ein pharmazeutisch unbedenkliches Säureadditionssalz davon enthält und im wesentlichen frei von dem entsprechenden (linksdrehenden) S-Isomeren ist.

3. Pharmazeutisches Antianginapräparat nach Anspruch 2 zur Verabreichung an ältere Patienten, Patienten mit hypokaliämischen Zuständen oder Patienten, die gleichzeitig nicht $K^+$-sparende Diuretika erhalten.

4. Verwendung einer Verbindung nach Anspruch 1 zur Herstellung eines Arzneimittels zur Behandlung von angina pectoris.

5. Verwendung einer Verbindung nach Anspruch 1 zur Herstellung eines Arzneimittels zur Behandlung von angina pectoris in Patienten mit niederem Kaliumgehalt im Blut oder Patienten, die gleichzeitig nicht $K^+$-sparende Diuretika erhalten.

**Revendications**

1. L'isomère R (dextrogyre) de formule:

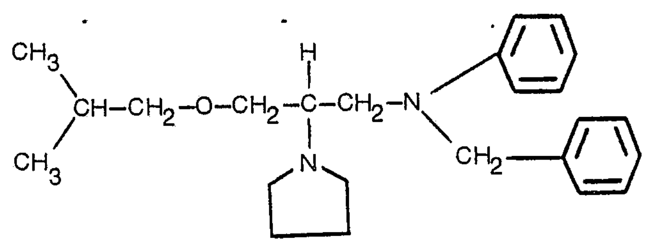

I

et ses sels d'addition d'acides convenant en pharmacie, qui sont essentiellement dépourvus de l'isomère S (lévogyre) correspondant.

2. Une préparation pharmaceutique contenant comme principe actif l'isomère R (dextrogyre) décrit dans la revendication 1 ou un de ses sels d'acides contenant en pharmacie, laquelle préparation est essentiellement dépourvue de l'isomère S (lévogyre) correspondant.

3. Une préparation pharmaceutique antiangoreuse selon la revendication 2 pour l'administration à des patients âgés, des patients en état hypokaliémique, ou des patients utilisant comme médicaments associés des diurétiques n'épargnant pas $K^+$.

4. L'emploi d'un composé selon la revendication 1 pour la fabrication d'un médicament pour le traitement de l'angine de poitrine.

5. L'emploi d'un composé selon la revendication 1 pour la fabrication d'un médicament pour le traitement de l'angine de poitrine chez des patients ayant de faibles taux sanguins de potassium ou des patients utilisant comme médicaments associés des diurétiques n'épargnant pas $K^+$.